(19) **Europäisches Patentamt**

**European Patent Office**

**Office européen des brevets**

(11) **EP 0 760 223 A1**

(12) **EUROPEAN PATENT APPLICATION**

(43) Date of publication:
05.03.1997 Bulletin 1997/10

(51) Int. Cl.⁶: **A61B 5/00**, G01N 21/31

(21) Application number: 95113654.8

(22) Date of filing: 31.08.1995

(84) Designated Contracting States:
DE FR GB

(71) Applicant: **Hewlett-Packard GmbH**
**D-71034 Böblingen (DE)**

(72) Inventor: **Kästle, Siegfried**
**D-71154 Nufringen (DE)**

(74) Representative: **Kurz, Peter**
**Hewlett-Packard GmbH,**
**Europ. Patent- und Lizenzabteilung,**
**Herrenberger Strasse 130**
**71034 Böblingen (DE)**

(54) **Apparatus for monitoring, in particular pulse oximeter**

(57) An apparatus for measuring medical parameters of a patient by means of transmission or reflection of electromagnetic, particularly optical waves, uses sinusoidal functions for demodulation in a multiplier (47,48). If at least two emitted waves are used, the modulation signals for the two waves are advantageously 90° shifted in phase with respect to each other. The apparatus is preferably a pulse oximeter.

FIG. 8

**Description**

FIELD OF THE INVENTION

This invention relates to an apparatus for measuring medical parameters of a patient by irradiation of electromagnetic waves into a sample or material and for measurement and subsequent analysis of the electromagnetic waves which have passed through said sample or material, and to a related method. More specifically and in one particular embodiment, the invention relates to the field of pulse oximetry.

BACKGROUND OF THE INVENTION

Optical methods for measuring medical parameters of a patient are well-known in the art. These include, for example, blood flow measurement, measurement of the perfusion, blood gas analysis, infrared gas analyzers etc., and in particular pulse oximetry. What is common to all of these methods is that light of the visible or adjoining spectra (such as infrared), or other electromagnetic waves, are irradiated into the tissue of a patient, into a sample taken from a patient (such as a blood sample) or into air inspired or expired by the patient. The light transmitted through, or reflected by such sample or material is then analyzed for changes of its characteristics, such as absorbance, wavelength etc., in order to determine a medical parameter of interest. It is understood that such methods are not limited to the use of electromagnetic waves of optical nature (i.e.,in the visible spectrum), but that they may use other waves in the electromagnetic spectrum as well. Further, such methods may use the *in vivo,* as well as the *in vitro* approach, i.e., they may be focused on the analysis of the patient (e.g., its tissue), or on samples taken from a patient.

In order to increase the clarity and comprehensibility of this description, the underlying problems will now be explained by means of one specific example, namely pulse oximetry. However, it is understood that this does by no means limit the scope of the present invention which is, in fact, also applicable for the measurement of other medical parameters.

Pulse oximetry is a non-invasive technique to evaluate the condition of a patient. Usually, a sensor or a probe comprises light emitting means such as light-emitting diodes (LEDs). Two or more of these LEDs with different wavelengths (e.g., red and infrared) may be used. The emitted light is directed into the tissue of the patient, and light receiving means such as photodiodes or phototransistors measure the amount of transmitted or reflected light. In the case of transmission measurement, the transmitter and receiver diodes are arranged opposite to each other with respect to the human tissue, whereas in the case of reflection measurement, they are arranged on the same side of the tissue.

The measured intensity can be used to calculate oxygen saturation in the arterial blood of the patient if measured at least at two wavelengths. The mathematical background therefor, which makes use of Lambert-Beer's law, has been described in sufficient detail in a multiplicity of former publications. See, for example, EP-A-262 778 which contains a rather good breakdown of the theory.

Usually, the sensor detachably connected to the pulse oximeter comprises at least two LEDs emitting light of a wavelength of e.g. 660 nm (Nanometers) - red - and 900 nm - infrared. The intensity of the emitted light can be modulated by the oximeter in that the exciting current of the LEDs is varied. The photocurrent received by the receiving element is measured by the oximeter and used to calculate the oxygen saturation of the arterial blood.

In today's oximeters, a hardware circuit contained in the front-end portion of an appropriate monitor makes use of a time multiplex approach, where the LEDs are switched on and off one after the other (see US 4,407,290; US 4,523,279 or US 3,847,483). The train of pulses usually consists of a minimum of 3 phases: an active red, a active infrared and a dark phase, where the ambient light is measured during the latter phase. Actually, there can be more than 3 phases to allow for more LEDs to be powered in one multiplexing time frame or additional dark phases. The phases are often of similar duration. The modulation frequency (repetition rate of the whole frame) ranges e.g. from 200 Hz to 2 kHz. This frequency should not be mixed up with the frequency of the light emitted by the LED's. In fact, the common modulation method uses pulse trains with pulses of rectangular shape to excite the LEDs, wherein the first pulse excites the red LED, the second pulse the infrared LED, and an interval without excitation is used to measure the ambient light (called "dark phase" in this context).

It has turned out, however, that such known oximeters, although widely used today in clinical practice (simply because they offer a non-invasive technology), provide limited performance only and, in particular, limited measurement accuracy. This happens mainly when sources of interference, such as neon lamps, UV lamps and other emitters of light, influence the optical path between the LEDs and the photoreceiver. The susceptibility of prior art oximeters to such sources of interference has been known by skilled people; however, no attempts have been made so far to evaluate the reasons therefor. In fact, the following considerations (which explain the mechanism of distortion of oximetry signals) reflect already the inventors thoughts on this problem, which in turn led him to the present invention, and can insofar already be considered as forming a part of, or - to some extent - the basis of the invention.

The frequency spectrum of a time multiplex signal (as described above) at the receiving photo diode consists of a couple of elements. The first is, of course, the spectral line of the LED modulation frequency. Other spectral lines of reduced amplitude appear at the harmonica of the basic modulation frequency, due to the fact that the spectrum of a pulse sequence contains spectral lines at multiples of the pulse repetition frequency. Such harmonics of significant amplitude appear up to an order of several tens.

Further, all spectral lines are not sharp lines, but rather broadened to some extent, such that they cover small bands in the spectrum. This is due to the fact that the signals are further modulated by the variation of the blood pulse (which contains components up to approx. 10 Hertz). This variation of the blood pulse is actually of interest for medical monitoring. Its frequency may be used to derive the patient's heart rate, and its amplitude is actually required to calculate oxygen saturation.

The blood pulse therefore broadens the spectral lines of the modulation frequency and its harmonics for about $\pm$ 10 Hertz. This is also called "physiological bandwidth", due to its origin in the physiological signal.

The details and drawbacks of the prior art technology will now be discussed with reference to the drawings.

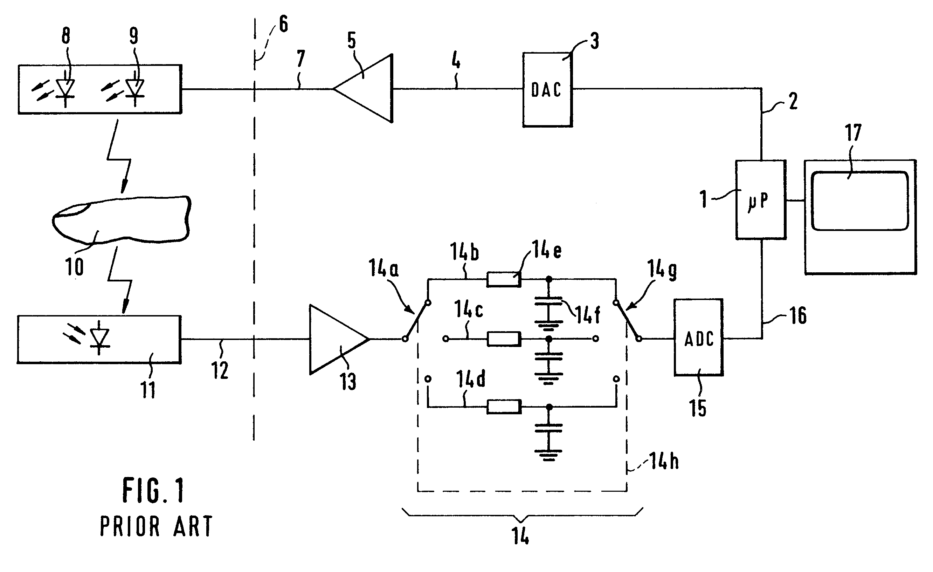

Fig. 1 depicts the essential functional blocks of a prior art oximeter. A microprocessor 1 controls operation of the oxygen saturation parameter. It generates digitally represented pulses for excitation of the LEDs. These are fed, via line 2, to digital-to-analog converter 3 which outputs analog signals of rectangular shape and feeds them, via line 4, to amplifier 5.

Dotted line 6 represents schematically the interface between the monitor and the sensor. That is, line 7 is physically a cable which connects the sensor and the monitor, and all elements left to dotted line 7 are in practice incorporated into the sensor. The sensor contains at least 2 LEDs 8 and 9 which emit light into the tissue of a patient, here a finger 10. Light transmitted through finger 10 reaches photodiode 11 and is fed, via line 12, to another amplifier 13. The amplified analog signal produced by amplifier 13 is fed (line 14) to a demodulation and filter section 14. This demodulation section comprises a demultiplexer 14a which feeds the amplified signal, depending on its time slot, to three different paths 14b to 14d which implement a low-pass/sample and hold function each. Demultiplexer 14a is controlled such that signals received during operation of LED 8 are fed to path 14b, signals received during operation of LED 9 are fed to path 14c, and signals received during the dark or ambient phase (LED's 8 and 9 switched off) are fed to path 14d.

Each of paths 14b to 14d operates as an independent low-pass filter - see, e.g., resistor 14e and capacitor 14f in path 14b -; the capacitor acts insofar also as a sample and hold device. The three paths 14b to 14d are then combined again by multiplexer 14g (which is synchronized with demultiplexer 14a, see dotted line 14h), and the signals are fed to analog-to-digital converter (ADC) 15. This ADC samples the incoming signals, typically once per channel, but a higher sampling rate is also possible. In other words, if a pulse is sent to the red LED 8, it is sensed once by ADC 15 to determine its amplitude, and the variation of the amplitude in succeeding pulse trains reflects pulsate variation of the arterial blood.

The digitized signal reaches, via line 16, microprocessor 1 which performs the necessary calculations to determine oxygen saturation. The results are then displayed on a display 17.

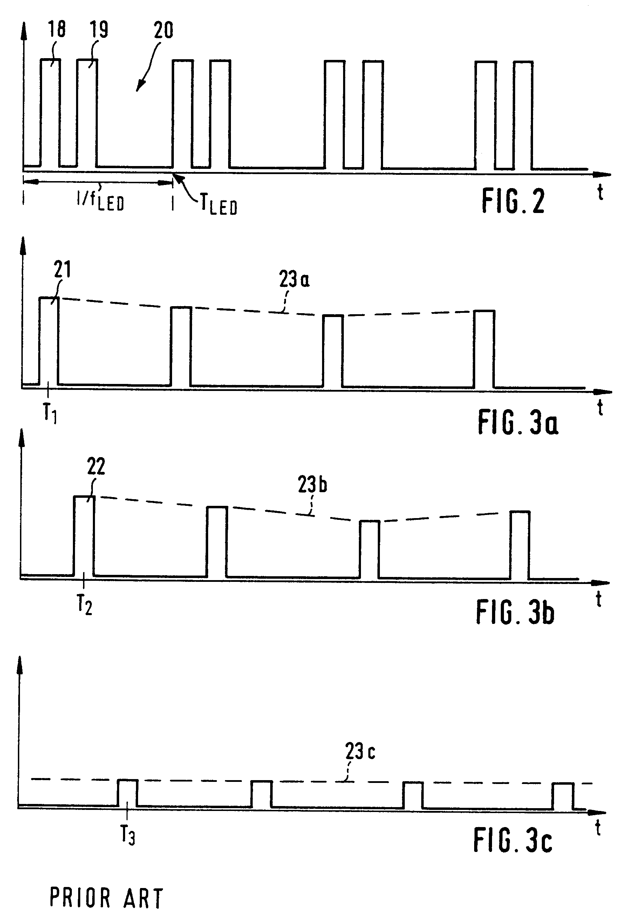

Fig. 2 is a timing diagram of the pulse train used to excite LEDs 8 and 9. A first pulse 18 controls red LED 8 (Fig. 1), and a second pulse 19 infrared LED 9. When both LEDs have been excited in sequence, a dark phase 20 is provided which is used on the receiver side to measure the amount of ambient light. At $t=T_{LED}$, the whole pattern starts again from the beginning. Thus, the modulation frequency is defined by

$$f_{LED} = \frac{1}{T_{LED}} \tag{1}$$

Figs. 3a to 3c depict the same pattern at the receiver side, separated according to paths 14b to 14d (Fig. 1), i.e., when the pulses have passed the human tissue. Fig. 3a shows the pulse of the red LED 8 (path 14b), Fig. 3b the pulse of the infrared LED 9 (path 14c), and Fig. 3c the signal detected on path 14d (ambient light). One will note that the red and infrared pulses (Figs. 3a and 3b) are

1. attenuated by a more or less fixed amount which is caused by non-pulsating tissue (DC attenuation);

2. subject to different attenuation in the red and infrared channel, as shown by the different amplitudes of red pulse 21 and infrared pulse 22; and that

3. their amplitudes are modulated with a slow frequency shown by dotted lines 23a and 23b (the frequency is even exaggerated in Figs. 3a and 3b for the purpose of demonstration), i.e., their amplitudes vary slowly over time. This slow frequency - in the range of 1 to 10 Hertz - reflects the blood pulsation in blood vessels hit by the light emitted by LEDs 8 and 9 and carries the physiological information required to calculate oxygen saturation. It is therefore are called "physiological signal", and the associated frequency band "physiological bandwidth". It will also be noted that the superimposed physiological signal has a very small amplitude, as compared to the overall amplitude of the received pulses.

In contrast, the pulse of Fig. 3c which represents ambient light is not modulated by pulsating tissue (ref. no. 23c).

The pulse trains shown in Figs. 3a to 3c are fed, via multiplexer 14g (Fig. 1), to the input of ADC 15. According to the prior art approach, ADC 15 takes at least one sample of the received pulse at $t=T_1$, $t=T_2$ and $t=T_3$. These samples represent the amplitudes of the red pulse, the infrared pulse and the ambient light, respectively. The technique discussed here corresponds to a demodulation with a rectangular or square wave.

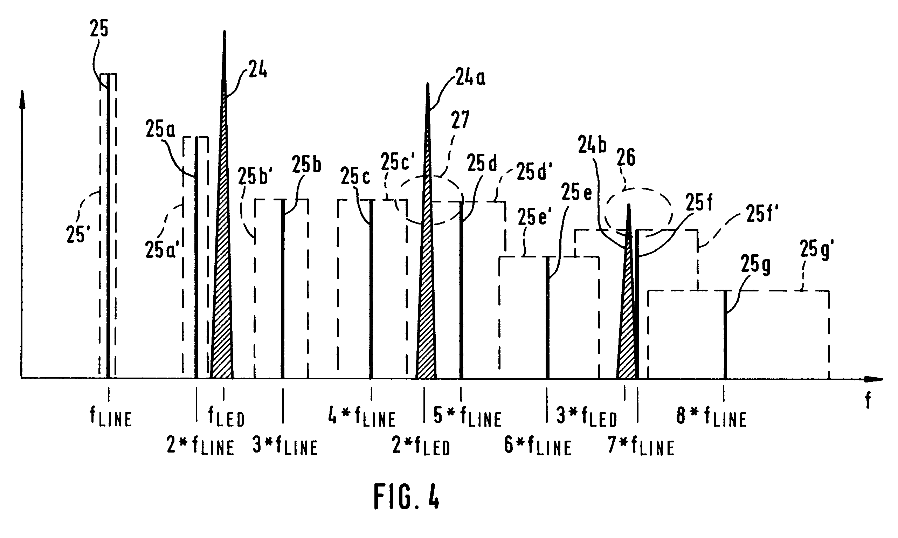

Now let us consider what happens in the frequency domain. Fig. 4 depicts the spectrum of the signal transmitted through the patient's tissue and amplified, but prior to demodulation and sampling, i.e., as it appears on line 14 (Fig. 1). The basic LED modulation frequency $f_{LED}$ is shown as spectral line 24. This is not a "sharp" spectral line. Instead, it is slightly broadened, due to the physiological signal (as discussed above) which in turn modulates the LED signal. The effect is that the LED modulation spectral line appears in fact as a small-band spectrum with a bandwidth of approx. $\pm$ 10 Hertz around the center of its base frequency.

Reference numbers 24a and 24b designate harmonics of the basic LED modulation freqency, i.e., $2*f_{LED}$ and $3*f_{LED}$. It is understood that these harmonics appear also broadened by the physiological bandwidth, i.e., they are centered at $\pm$ 10 Hertz around the corresponding harmonic frequency.

In contrast, reference number 25 represents the spectral line of the mains (power line) - at $f_{Line}$ - which is the major source of interference; typically, at 50 Hertz (Europe) or 60 Hertz (United States). It is understood that, although the power line has always a specific frequency, this frequency is subject to variations (however small and slow) of the mains frequency. This variability or tolerance band is indicated by dashed box 25'.

The spectrum also contains harmonics of the basic mains frequency $f_{Line}$. These are denoted as 25a through 25g in Fig. 4 and represent frequencies of $2*f_{Line}$ to $8*f_{Line}$ (it will be appreciated that harmonics of even higher order do also exist, but have not been drawn in Fig. 4). Like the basic mains frequency, their harmonics are also spread, see dotted boxes 25a' through 25g'. The diagram shows that the higher the order of the harmonics, the higher the possible variations in frequency.

Fig. 4 reveals also another effect. That is, some of the harmonics of the LED modulation frequency $f_{LED}$, and of the mains frequency $f_{Line}$, are quite close to each other. A typical example is shown by reference number 26. The third order harmonics 24b of the LED modulation frequency, and the seventh order harmonics 25f of the mains frequency, are quite close to each other ($3*f_{LED} \approx 7*f_{Line}$). This means that there is frequent interference in the related bands, in particular in consideration of the broad tolerance band 25f' associated with the seventh order harmonics 25f of the mains frequency. But there are also other doubtful cases, such as indicated by dotted circle 27. Although there is in fact some distance between the second order harmonics 24a of the LED modulation frequency and the fifth order harmonics 25d of the mains frequency, there is still an overlap if we consider the whole tolerance band 25d' associated with $5*f_{Line}$. In other words, if the frequency of the power line deviates slightly from its nominal value, its fifth order harmonic might approach the second order harmonic of the LED modulation frequency, such that there is at least sporadic interference. If we proceed to higher order harmonics, there will always be a danger of interference, just because the tolerance band associated with the harmonics of the mains frequency becomes broader and broader, such that the associated spectra overlap. This is shown by spectra 25e', 25f' and 25g'. It is evident that for these higher frequencies, any harmonic of the LED modulation frequency will necessarily fall into at least one bandwidth of the harmonics of the mains frequency.

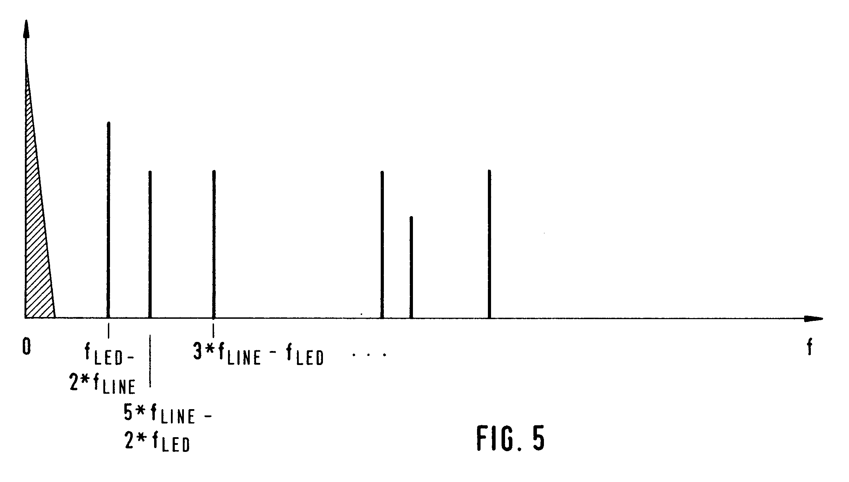

One might now ask the question how such interference of the harmonics influences the base band and thus the results of oxygen saturation measurement. The underlying mechanism is demonstrated in Fig. 5.

According to the prior art approach, the received signal is demodulated by demultiplexer 14a. (This kind of demodulation corresponds to synchronous AM demodulation with a square wave, as e.g. described in US 4,807,630). Demodulation with a square wave has the effect that all kinds of difference and sum frequencies- in particular, of the harmonics - appear in the base band close to the signal of interest; i.e., the harmonics are folded down into the base band. This effect is, by way of example, illustrated in Fig. 5. This figure is based on the understanding that the LED sampling frequency is equal to the LED modulation frequency. (Every channel - red, infrared, dark - is separately sampled and demodulated).

However, the harmonics of the mains frequency are only one source of interference which may distort the useful signal for determining oxygen saturation. In a clinical environment, the pulse oximetry sensor picks up ambient light and various electromagnetic noise. The major source for ambient light is room illumination with fluorescence ceiling lamps which gives broad spectral bands with harmonics at harmonica of the power line frequencies, typically 50 Hertz or 60 Hertz. However, electrical noise also comes very often from the power line and shows up as harmonica of the mains frequency. Other well known sources for largely interfering electrical noise are electro-surgery devices used in the operating rooms. They can be very broad-band and at any frequency.

As explained above, the spectra of the signal at multiples of the LED modulation overlap very likely with the spectra of the optical or electrical noise components. Any noise lines in one of the LED modulation bands will be demodulated and intrinsically folded down to the base band and contribute to poor signal-to-noise ratios (S/N). A very dangerous situation for the patient can occur in the monitoring of neonates. These are often treated with very bright UV lamps for the bilirubin photo therapy. As they produce poor signals because of a poor vascular perfusion, the amount of ambient light can cause even situations with a signal-to-noise ratio < 1. A pulse oximeter is very likely to be mislead in these situations. It can derive values for pulse rate, oxygen saturation and perfusion index which are wrong because the input signals are dominated by noise instead of patient signals.

On the basis of these thoughts and considerations, it is a major objective of the present invention to provide an apparatus for measuring medical parameters of a patient by irradiation of electromagnetic waves into a sample or material and for measurement and subsequent analysis of the electromagnetic waves which have passed through the sample or material, the analysis producing an indication of the patient's physical condition, wherein the apparatus comprises:

- A source of a modulation signal,
- at least one emitter for electromagnetic waves, said emitter being controlled by the modulation signal,
- at least one receiver for electromagnetic waves which have passed through the sample or material,
- a demodulator for demodulating a signal representative of the electromagnetic waves received by the receiver,

and specifically a pulse oximeter, which is less susceptible to electromagnetic interference and thus provides better performance and measuring accuracy even under unfavourable conditions, specifically under the influence of a multiplicity of sources of electromagnetic (in particular, optical) noise in a clinical environment.

SUMMARY OF THE INVENTION

The basic idea of the present invention may be characterized in that the inventor has recognized, for the first time, the disadvantages of the time multiplex demodulation technique used in all common pulse oximeters so far. This led him to the conclusion that a completely different approach might be useful, although all skilled people still were of the opinion that the time multiplex approach was the only one suited for this kind of measurement, and designed oximeters accordingly.

In general, it is the major finding of the invention that, in an apparatus for medical monitoring as discussed supra, a demodulator may be used which includes a multiplier receiving the signal representative of the electromagnetic waves received by the receiver at one input, and a sinusoidal signal with a frequency corresponding to the frequency of the modulation signal at another input, wherein the analysis is based on the output signal of the demodulator. In other words, the demodulation of the signal showing up at the receiver is no longer performed by means of a square wave, at is has been practice now for about 20 years, but rather with a signal of sine-wave shape. It can be shown (see detailed description) that this approach avoids that harmonics of the sources of noise, in particular of the power lines or related optical or other electromagnetic interference, are folded down into the base band and thus impact the signal-to-noise ratio. The result is thus a more reliable value, in particular an absolute value of high performance (in contrast to the prior art, wherein merely the trend was really meaningful). The invention therefore overcomes a prejudice of the skilled people in the design of medical instrumentation, with all of the related advantages. The phase of sinusoidal demodulation signal preferably corresponds to the phase of the modulation signal, corrected by the system phase shift.

It is understood that the finding of the invention may be applied to all kinds of medical measurement wherein electromagnetic waves are irradiated into biological material, and wherein the waves which have passed through said biological material are demodulated and analyzed for the purpose of determining medical parameters ("passed" in the sense used herein covers transmitted, as well as reflected waves). Examples of such measurements are impedance pneumography or infrared gas analyzers. However, these examples also demonstrate that the investigated material need not be biological material in the classical sense, and that the application of the invention is not restricted to *in vivo* measurements. In contrast, it covers *in vitro* methods as well, such as conventional blood gas analyzers, and even other substances than biological substances in the classical meaning, such as air and gases expired by the patient. All of this is covered by the term "sample or material" as used herein.

However, the preferred application of the present invention is in the field of optical measurement (wherein waves in the optical spectrum are considered as a special case of electromagnetic waves), and in particular pulse oximetry.

It is further understood that the invention is mainly incorporated in the demodulation section of a medical measuring apparatus, and is thus embodied in a medical monitor, even if no sensors are connected to it. Therefore, the invention also covers an apparatus for measuring medical parameters of a patient by irradiation of electromagnetic waves into a sample or material and for measurement and subsequent analysis of the electromagnetic waves which have passed through the sample or material, said analysis producing an indication of the patient's physical condition, wherein the apparatus comprises:

- A source of a modulation signal for controlling an emitter of electromagnetic waves,
- a demodulator for demodulating a signal representative of electromagnetic waves which have passed through the sample or material as received by a receiver,

wherein the demodulator includes a multiplier receiving the signal representative of the electromagnetic waves received by the receiver at one input, and a sinusoidal signal with a frequency and a phase corresponding to the frequency and phase of the modulation signal at another input, and said analysis is based on the output signal of the demodulator.

Specifically in the case of optical measurement, and again particularly for pulse oximeters, a second optical channel may be provided. In this case, the apparatus according to the invention includes a second source of a second modulation signal for controlling a second emitter of electromagnetic waves, and a second demodulator for demodulating a signal representative of electromagnetic waves which have passed through the sample or material received by a receiver, wherein the second demodulator includes a second multiplier receiving the signal representative of the electromagnetic waves received by the receiver at one input, and a second sinusoidal signal with a frequency corresponding to the frequency of the second modulation signal at another input, and said analysis is based on the output signals of the demodulator and the second demodulator. The wording used herein cites a first and second demodulator; it will be understood, however, that their functionality may also be integrated in a single operating component of the monitor. Specifically, a single hardware component may be provided which incorporates both channels in program implementation. Similar considerations apply to the receiver; although a common receiver may be used for both channels, there may also be separate receivers. Further, it is also possible to provide more than two channels.

In the case of two or more channels, it is most preferable if

- first and second sources of modulation signals generate modulation signals of essentially the same frequency,
- the first and second modulation signals are shifted in phase for a fix amount with respect to each other, and
- the first and second sinusoidal signals provided to the first and second multiplier are also of the same frequency as the modulation signals and shifted in phase for the same fix amount with respect to each other.

This approach has the outstanding advantage that the harmonics of the spectral lines of the useful signal can be completely separated from the harmonics of noise, as will be later shown in the detailed description. The most preferable solution is a phase shift of 90°; however, other phase shifts may be used as well. In the latter case, there will be some cross-influence between the channels, but this can be compensated for by appropriate mathematical algorithms.

One alternative solution for the 90° phase shift is to use different modulation frequencies for the various channels.

Another essential feature of the present invention is that the frequency of the modulation signals is approximately f=275 Hertz. This selection guarantees that the main spectral line of the modulation frequency is placed optimally between harmonics of the noise bands for 50 Hz and 60 Hz mains frequency, even if there is a variation or instability in the mains frequency (see detailed description). Therefore, this selection of the modulation frequency further contributes to a noise-free, reliable measurement.

The modulation signals themselves may be sine waves, in which case the mathematical theory becomes particularly easy. However, square or rectangular waves may be used as well. Square waves are easy to generate and have another related advantage: That is, the duty cycle may be easily varied without changing the fundamental frequency, in order to adapt the signal strength in various channels. (The shape of the modulation signals should not be mixed up with the signals used for demodulation; according to the invention, the latter have always to be sinusoidal signals).

In a preferred embodiment of the invention, a low pass filter is provided through which the output signal of the multiplier circuit is fed. Likewise, it is advantageous to use a bandfilter connected between the receiver of the electromagnetic waves and the demodulator. This bandfilter blocks the harmonics, and even the fundamental frequencies of any noise, and further operates as an anti-aliasing filter for subsequent analog-to-digital conversion.

As already mentioned, one major application of the invention is pulse oximetry. In this case, the biological material examined *in vivo* is the patient's tissue. The electromagnetic waves are preferably waves selected from the visible and the adjoining spectra of light, in particular red and/or infrared light. The emitters of light are advantageously light-emitting diodes which are of small size and easy to incorporate into a sensor.

The invention also relates to a method for measuring medical parameters of a patient by irradiation of electromagnetic waves into a sample or material and for measurement and subsequent analysis of the electromagnetic waves which have passed through said sample or material, wherein said method comprises the steps of:

- Generating a modulation signal,
- irradiating electromagnetic waves into the sample or material under control of the modulation signal,
- receiving electromagnetic waves which have passed through the sample or material,
- demodulating a signal representative of the received electromagnetic waves by feeding it to a multiplier and multiplying it with a sinusoidal signal of the same frequency as the modulation signal, such as to generate a demodulated signal, and
- analysing the demodulated signal.

Again, it is possible to expand this concept to a second, a third etc. channel, and to choose a 90° phase shift (or any other fixed amount of phase shift) for the modulation signals.

Just another aspect of this invention is that the phase shift of an apparatus for medical monitoring may be continuously adjusted (even if a measurement cycle is running). Such adjustment comprises the steps of:

- Disabling the second modulation signal,
- applying a default phase $\varphi_{def}$ for demodulation,
- monitoring the first and second demodulated signals $L_1$ and $L_2$,
- calculating the correct system phase

$$\varphi = \varphi_{def} + \arctan(L_1/L_2),$$

- applying the correct system phase $\varphi$ for demodulation,
- enabling the second modulation signal.

and provides a precise indication of the phase shift effected by apparatus, such as to enable an "autozero" function for the phase.

BRIEF DESCRIPTION OF THE DRAWINGS

The invention will now be explained, by means of a non-limiting example, with reference to the accompanying drawings, in which:

| | |
|---|---|
| Fig. 1 | depicts a block diagram of a prior art oximeter, |
| Fig. 2 | is a timing diagram of the pulse train emitted by a prior art oximeter, |
| Figs. 3a to 3c | are timing diagrams of the pulse trains received by a prior art oximeter. |
| Fig. 4 | is the spectrum of a prior art time multiplex oximeter prior to demodulation, |
| Fig. 5 | depicts the effect of demodulation on the spectrum in prior art oximeters, |
| Fig. 6 | is a block diagram of a pulse oximeter according to the present invention, |
| Fig. 7 | depicts the shape and timing of the pulses used to control the light-emitting diodes, |
| Fig. 8 | is a block diagram of the digital signal processor, |
| Fig. 9 | is an example of a spectrum processed by an apparatus according to the present invention, |
| Fig. 10 | shows the effect of the quadrature modulation technique according to the present invention on the signal-to-noise improvements, and |
| Fig. 11 | is a vector diagram for illustrating phase shift measurement. |

DETAILED DESCRIPTION OF THE PREFERRED EMBODIMENTS

Figs. 1 through 5 illustrating the prior art approach have already been discussed above, such that there is no need to consider them further.

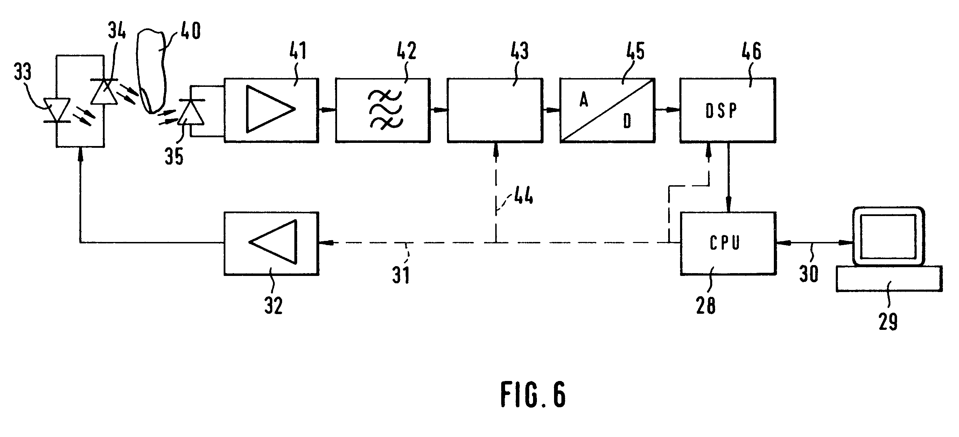

Fig. 6 depicts a block diagram of a two-wavelength pulse oximeter according to the invention. A microcontroller or CPU (central processing unit) 28 controls overall operation of the oximeter. In particular, it starts the measurements, adjusts the gain and controls the timing of LED excitation. Further, most of the signal processing, in particular calculation of the pulse oximetry values (oxygen saturation), of the pulse rate, the perfusion indicator and plethysmograhic waveforms is performed by CPU 28. Such algorithms - specifically suited for artifact suppression - are, for example, described in another patent, US 5,299,120, originating from the inventor of the present case. CPU 28 may further communicate with other equipment, such as a host monitor 29, via a digital link 30. The host monitor is equipped with data and waveform display capabilities, controls the alarm limits etc. It is, however, understood that the display of data, manual input etc. could also be performed locally. Further, CPU 28 may even communicate with larger systems such as hospital information systems or central stations.

The control signals for driving, i.e.,for switching the LEDs are fed from CPU 28, via line 31, to an LED driver circuit 32 (the digital-to-analog converter is not shown here and may be integrated in CPU 28). LED driver circuit 32 contains current sources which operate light emitting diodes (LEDs) 33 and 34 in switched mode.

In Fig. 6, a red LED is designated as 33, and an infrared LED as 34. Both LEDs, as well as a photodiode 35, are integrated into an appropriate sensor. Such sensors are well-known in the art and need not be discussed in detail here. Reference is made, for example, to DE-C-37 03 458. The rest of the elements shown in Fig. 6 are not incorporated in the sensor, but in an appropriate monitor, a signal pick-up box or the like.

LED driver circuit 32 operates LEDs 33 and 34 in antiparallel mode. That is, red LED 33 is operated by pulses of one polarity, and infrared LED 34 is operated by pulses of the opposite polarity. This design requires less connections between the sensor and the associated monitor, although it is not a necessary prerequisite for practising the present invention.

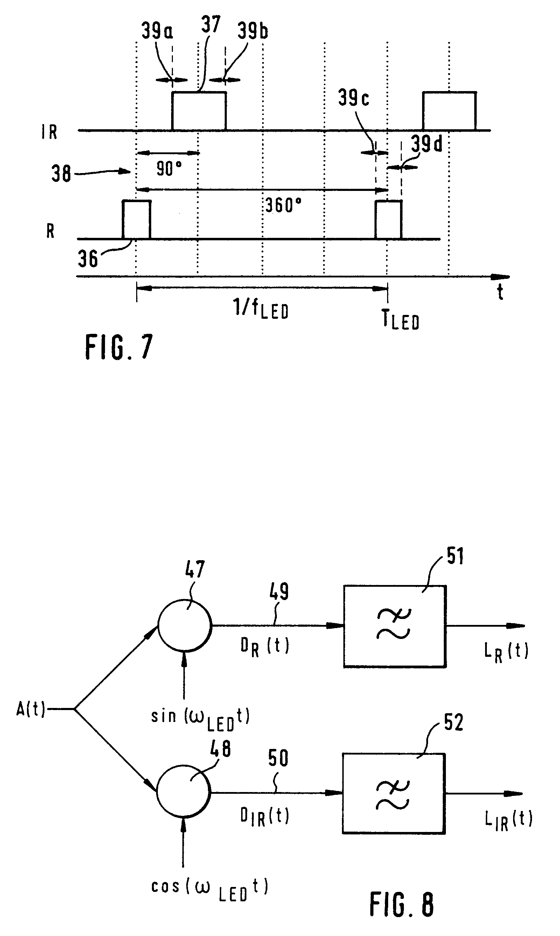

The timing of the pulses generated by LED driver circuit 32 will now be discussed with reference to Fig. 7. In the shown example, these pulses are of rectangular shape, although they could also be shaped as a sine, or incorporate any other suitable shape.

The pulse driving the red LED 33 is designated as 36. Another pulse 37 (of opposite polarity, not shown in Fig. 7) drives infrared LED 34. The whole pattern restarts at $t=T_{LED}$, such that a time interval of $T_{LED}$ defines a complete cycle of 360°. An important design feature of the timing chosen in the pulse oximeter according to the invention is that the pulses of the red and the infrared LED, respectively, are offset by 90°, see reference number 38. (In terms of timing, this phase shift corresponds to $T_{LED}/4$). The reference lines for the 90° phase shift are the center of the red and infrared pulse, respectively. This is of importance if a duty cycle adjustment is necessary or desirable to balance the red and infrared signals. Such duty cycle adjustment means to adapt the pulse length of either or both of the pulses, as indicated by arrows 39a to 39d.

Returning now to Fig. 6, the light pulses generated by LEDs 33 and 34 pass human tissue, as indicated by a finger 40, and are received by photodiode 35. The output signal of the photodiode is fed to a photo amplifier 41. This photo amplifier converts the photo current of the photodiode into a voltage. In contrast to prior art time multiplex systems, its bandwidth can be limited to $f_{LED}$ in the present invention. Its gain is selected as high as possible, but within the limits of the maximum expected photo currents.

Reference number 42 designates a band filter. The main purpose of that stage is to block out-of-the band noise and to serve as an anti-aliasing filter for analog-to-digital sampling. The passband is designed as narrow as possible around the center frequency $f_{LED}$. The bandwidth is only slightly broader than the bandwidth of the physiological blood pulse signal. Any harmonics of the LED sampling frequency can be blocked out as they do not contribute to the demodulated signal.

Programmable gain amplifier 43 (controlled by CPU 28, see line 44) ensures that the filtered signal fits within the input range of analog-to-digital converter (ADC) 45. It is therefore used to adapt to the large dynamic variability of the photoelectrical signal. As pulse oximetry sensors are used on very different body locations, the different tissue types and thickness between emitters and receivers result in largely different light intensities. The gain of that amplifier is controlled by the CPU to adapt the output amplitude to fit the full scale input range of the analog-to-digital converter for good quantization.

Digital signal processor (DSP) 46 represents, to some extent, the "heart" of the present invention. It demodulates the received, filtered and digitized pulses with sinusoidal functions. In the shown embodiment, this demodulation is performed in the digital domain - i.e., by a digital processor -, and in fact the functionality of DSP 46 may be provided by CPU 28 as well. However, this is not a necessary requirement; DSP 46 may also be a separate signal processor in digital or analog technology. The major functions of DSP 46 are demodulation, low pass filtering and down sampling.

In order to explain the functionality provided by DSP 46, reference is now made to Fig. 8. The output signal of ADC 45, A(t), is fed to two multipliers 47 and 48, which perform a multiplication with sinusoidal functions $\sin(\omega_{LED}t)$ and $\cos(\omega_{LED}t)$, respecitvely. The skilled man will immediately note that the use of sine and cosine functions corresponds to a phase shift of 90°, just because $\sin(90°+\alpha) = \cos(\alpha)$. The demodulated signals are now fed, via lines 49 and 50, to respective digital low pass filters 51 and 52. Their functionality will be discussed in greater detail below.

For the following discussion of the mathematical theory, it will be assumed that the LEDs are driven so that the optical emissions of the LEDs are pure sine waves, shifted by 90° in phase, e.g. the red LED with a sine and the infrared LED with a cosine. In the actual embodiment, this is not the case, as shown above; however, the mathematical background may be explained more easily by the assumption of sinusoidal driving.

The output of ADC 45, i.e., the input signal to DSP 46 is then

$$A(t) = A_R(t) + A_{IR}(t) \tag{2}$$

with

$$A_R(t) = A_R {}^* \sin(2\pi {}^* f_{LED} {}^* t + \varphi) \tag{3a}$$

$$A_{IR}(t) = A_{IR} {}^* \cos(2\pi {}^* f_{LED} {}^* t + \varphi) \tag{3b}$$

wherein $\varphi$ is the phase shift between the optical LED signal and the digitized photo receiver signal at the ADC output, and $\omega_{LED}=2\pi {}^* f_{LED}$.

$A_R$ and $A_{IR}$ themselves are modulated by the patient's blood pulse:

$$A_R = S_R(t) \tag{4a}$$

$$A_{IR} = S_{IR}(t) \tag{4b}$$

wherein $S_R(t)$ and $S_{IR}(t)$ are composed of a DC and an AC component. (The DC component represents the constant tissue absorption, whereas the AC component is related to the variable absorbance due to pulsatile blood volume change).

Demodulation as multiplication with sine waves reveals then at the outputs of multipliers 47 and 48

$$D_R(t) = A(t)^* \sin(\omega_{LED}{}^*t + \varphi) \tag{5a}$$

$$D_{IR}(t) = A(t)^* \cos(\omega_{LED}{}^*t + \varphi) \tag{5b}$$

If we introduce the definition of A(t), we obtain

$$D_R(t) = \tfrac{1}{2}A_R - \tfrac{1}{2}A_R{}^* \cos(2\omega_{LED}{}^*t + 2\varphi) + \tfrac{1}{2}A_{IR}{}^* \sin(2\omega_{LED}{}^*t + 2\varphi) \tag{6a}$$

$$D_{IR}(t) = \tfrac{1}{2}A_{IR} - \tfrac{1}{2}A_{IR} \cos(2\omega_{LED}{}^*t + 2\varphi) + \tfrac{1}{2}A_R{}^* \sin(2\omega_{LED}{}^*t + 2\varphi) \tag{6b}$$

So far, the demodulated signals contain both the sum and difference frequencies. These signals are then fed through digital low pass filters 51 and 52 which have a cut-off frequency just beyond the physiological bandwidth of the blood pulse. In an ideal low pass filter, this operation cuts off all harmonics of the modulation frequency $f_{LED}$.

In this case, the output of low-pass filters 51 and 52 is

$$L_R(t) = \tfrac{1}{2} A_R \tag{7a}$$

$$L_{IR}(t) = \tfrac{1}{2} A_{IR} \tag{7b}$$

These signals correspond ideally to the optical absorption of the red and infrared signals at the photodiode. Note that signals $A_R$ and $A_{IR}$ are not constant, but are modulated by the blood pulsation itself.

Signals $L_R(t)$ and $L_{IR}(t)$ are now small-band signals, which means that the data rate is reduced to a level desirable by standard down-sampling techniques. It is further understood that all other frequencies in the received signal, like electrical or optical interference, are blocked completely by the method according to the present invention. The result is very pure, noise-free signal reconstruction. Therefore, it is possible to choose an interference-free modulation band, even at lowest frequencies. In the preferred embodiment of the present invention, the LED modulation frequency is selected as $f_{LED} = 275$ Hertz.

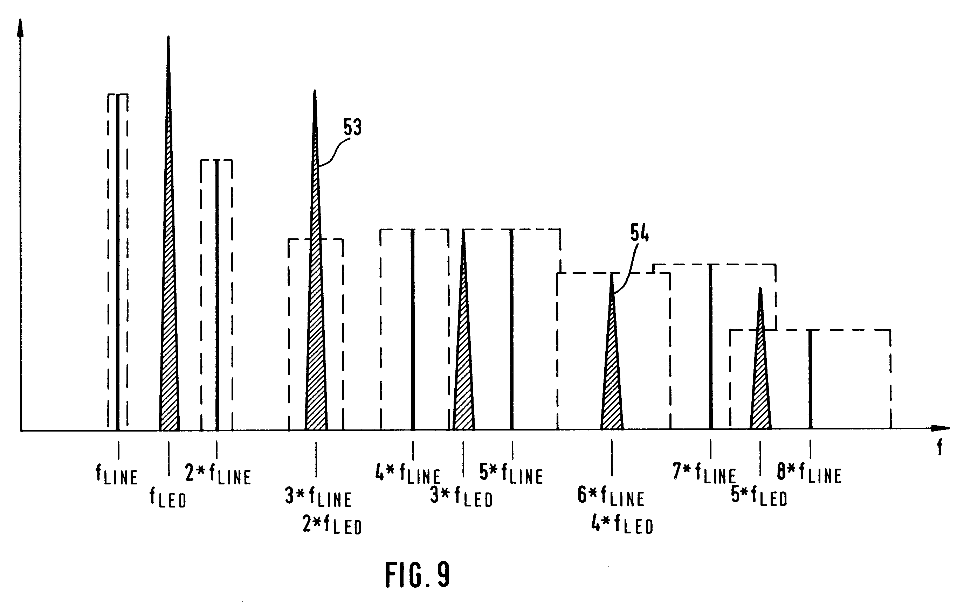

In order to explain the advantages of the present invention, as compared to the prior art time multiplex approach, and in particular the advantages of demodulation with sine waves, preferably with a phase offset of 90°, reference is now made to Fig. 9. This figures depicts a typical spectrum in a representation similar to Fig. 4. However, Fig. 9 depicts even a more sensitive case, just because some of the harmonics of the LED modulation frequency, and some harmonics of the mains frequency coincide. Reference numbers 53 and 54 relate to such cases:

$$2^* f_{LED} \approx 3^* f_{Line} \text{ (Ref. no. 53)} \tag{8a}$$

$$4^* f_{LED} \approx 6^* f_{Line} \text{ (Ref. no. 54)} \tag{8b}$$

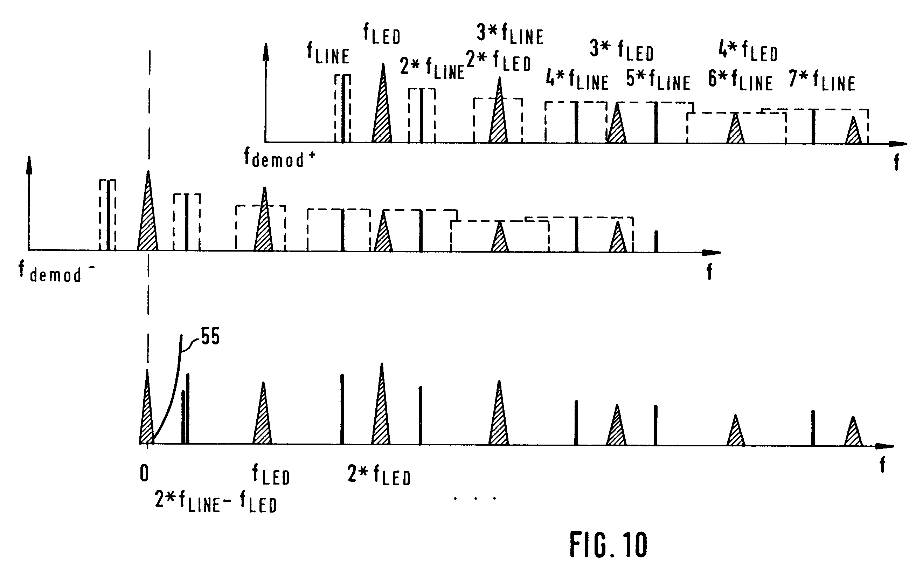

The demodulation technique according to the present invention is, however, still able to filter the harmonics of the mains frequency out, as will now be shown by means of Fig. 10.

The quadrature demodulation technique according to the present invention, in combination with sampling, produces a shift of the related spectra, together with the generation of sum and difference frequencies. The amount of frequency shift is $f_{demod}$, i.e.,the frequency of the sine functions used for demodulation, which is in this case identical to the LED modulation frequency $f_{LED}$. The shifted spectra (two in total) are shown in Fig. 10, namely the spectrum shifted by $+f_{demod}$ (upper diagram) and the spectrum shifted by $-f_{demod}$ (second diagram from top). (The frequency designations are, except of the shift identical in both diagrams, so they are only shown in the upper one).

The resulting spectrum is shown in the lower diagram. A low pass filters out the base band and blocks all higher frequencies (its attenuation characteristics is designated by reference number 55). As can be easily observed, the baseband signal does not contain any noise resulting from the harmonics of the power line or other sources of interference, which makes up the great advantage of the present invention over the prior art approach.

CPU 28 further performs a measurement of the phase shift ($\varphi$) of the whole system. ($\varphi$ is the phase difference between the excitation signals which drive each LED and the receiving signal as sampled by the A/D converter). A software program in CPU 28 performs that measurement routinely at power-up and at selected time intervals during normal operation. The procedure is as follows:

Step 1:    Interrupt normal operating mode; disable red LED; run infrared LED in normal mode.

Step 2:     Apply a default phase $\varphi_{def}$ in demodulator.

Step 3:     Wait for some milliseconds until low pass filters have settled.

Step 4:     Store the real-time values for $L_R(t) = L_R'$ and $L_{IR}(t) = L_{IR}'$.

Step 5:     Calculate the correct system phase $\varphi$.

Step 6:     Apply the correct system phase $\varphi$.

Step 7:     Enable the red LED.

Step 8:     Wait until low pass filters have settled.

Step 9:     Return to normal operating mode.

The calculation of the correct system phase shift $\varphi$ is performed by means of the following formula:

$$\varphi = \varphi_{Def} + \sigma \qquad\qquad (9)$$

wherein

$$\sigma = \arctan(L_R'/L_{IR}') \qquad\qquad (10)$$

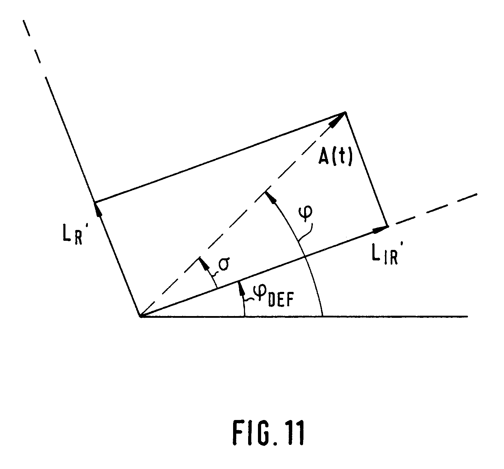

The meaning of these formulae is illustrated in Fig. 11, wherein $L_R'$ is the default red demodulation vector, $L_{IR}'$ is the default infrared demodulation vector, and $A(t) = A_{IR}(t)$, i.e., $A(t)$ contains only an infrared portion during phase calibration. $L_R'$ is the cross-coupled demodulation fraction from an infrared signal to the red channel prior to optimum phase adjust.

It will be appreciated by those skilled in the art that the principle of sinusoidal demodulation as disclosed herein is also applicable to multi-wavelength oximetry. If a third and forth LED is used, a further LED modulation frequency has to be selected. In general, for n LEDs, n/2 modulation frequencies have to be used, just because each frequency channel can contain 2 independent information data in quadrature mode. The only condition for these different modulation frequencies is that they are offset by more than the physiological bandwidth of the blood pulse.

It is also clear that the sinusoidal demodulation can be applied in a two LED pulse oximeter without the quadrature concept, but with only the sinusoidal demodulation, if there is a different modulation frequency chosen for each LED.

The great advantage of interference immunity of this system may also be applied to other sensoric measurements where ambient noise is an issue, not only in medical instrumentation. It is always possible where an excitation is used for stimulation. Examples are impedance measurements in bridges like pressure strain gauges or spectrometer devices with chopped sources.

## Claims

1.  Apparatus for measuring medical parameters of a patient by irradiation of electromagnetic waves into a sample or material and for measurement and subsequent analysis of the electromagnetic waves which have passed through said sample or material, said analysis producing an indication of the patient's physical condition, wherein said apparatus comprises:

    (1.1) A source of a modulation signal,
    (1.2) at least one emitter (33) for electromagnetic waves, said emitter being controlled by said modulation signal,
    (1.3) at least one receiver (35) for electromagnetic waves which have passed through said sample or material,
    (1.4) a demodulator (46) for demodulating a signal representative of the electromagnetic waves received by said receiver (35),

    wherein said demodulator (46) includes a multiplier (47) receiving said signal representative of the electromagnetic waves received by said receiver (35) at one input, and a sinusoidal signal with a frequency corresponding to the frequency of said modulation signal at another input, and wherein said analysis is based on the output signal of said demodulator (46).

2.  Apparatus for measuring medical parameters of a patient by irradiation of electromagnetic waves into a sample or material and for measurement and subsequent analysis of the electromagnetic waves which have passed through said sample or material, said analysis producing an indication of the patient's physical condition, wherein said apparatus comprises:

    (2.1) A source of a modulation signal for controlling an emitter (33) of electromagnetic waves,
    (2.2) a demodulator (46) for demodulating a signal representative of electromagnetic waves which have passed through said sample or material as received by a receiver (35),

wherein said demodulator (46) includes a multiplier (47) receiving said signal representative of the electromagnetic waves received by said receiver (35) at one input, and a sinusoidal signal with a frequency corresponding to the frequency of said modulation signal at another input, and wherein said analysis is based on the output signal of said demodulator (46).

3. Apparatus according to claim 2 and including a second source of a second modulation signal for controlling a second emitter (34) of electromagnetic waves, and a second demodulator (46) for demodulating a signal representative of electromagnetic waves which have passed through said sample or material received by a receiver (35), wherein said second demodulator (46) includes a second multiplier (48) receiving said signal representative of the electromagnetic waves received by said receiver (35) at one input, and a second sinusoidal signal with a frequency corresponding to the frequency of said second modulation signal at another input, and said analysis is based on the output signals of said demodulator (46) and said second demodulator (46).

4. Apparatus according to claim 3, wherein

(4.1) said first and second sources of modulation signals generate modulation signals of essentially the same frequency,
(4.2) the first and second modulation signals are shifted in phase for a fix amount with respect to each other,
(4.3) the first and second sinusoidal signals provided to the first and second multiplier (47,48) are also of the same frequency as the modulation signals and shifted in phase for the same fix amount with respect to each other.

5. Apparatus according to claim 4, wherein said fix amount of phase shift is 90°.

6. Apparatus according to claim 4, wherein the frequency of said first and second modulation signals is basically $f=275$ Hertz.

7. Apparatus according to claim 4, wherein said first and second modulation signals are square-wave signals.

8. Apparatus according to claim 7, wherein the duty cycle of said first and second modulation signals is adjustable.

9. Apparatus according to claim 4, wherein said first and second modulation signals are sinusoidal signals.

10. Apparatus according to claim 2, including a low pass filter (51,52) through which the output signal of said multiplier circuit (47,48) is fed.

11. Apparatus according to claim 2, including a bandfilter (42) connected between said receiver (35) for electromagnetic waves and said demodulator (47,48).

12. Apparatus according to claim 3, wherein said sample or material is said patient's tissue.

13. Apparatus according to claim 12, wherein said electromagnetic waves are waves selected from the visible and/or the adjoining spectra of light, preferably red and/or infrared light.

14. Apparatus according to claim 13, wherein said first and second emitters (33,34) of electromagnetic waves are light-emitting diodes.

15. Apparatus according to claim 14, wherein said apparatus is a pulse oximeter, and said medical parameter is oxygen saturation.

16. Method for measuring medical parameters of a patient by irradiation of electromagnetic waves into a sample or material and for measurement and subsequent analysis of the electromagnetic waves which have passed through said sample or material, said method comprising the steps of:

(16.1) Generating a modulation signal,
(16.2) irradiating electromagnetic waves into said sample or material under control of said modulation signal,
(16.3) receiving electromagnetic waves which have passed through said sample or material,

(16.4) demodulating a signal representative of the received electromagnetic waves by feeding it to a multiplier (47) and multiplying it with a sinusoidal signal of the same frequency as said modulation signal, such as to generate a demodulated signal,

(16.5) analysing said demodulated signal.

**17.** Method according to claim 16, including the steps of:

(17.1) Generating a second modulation signal,

(17.2) irradiating electromagnetic waves into said sample or material under control of said second modulation signal,

(17.3) receiving electromagnetic waves which have passed through said sample or material,

(17.4) demodulating a signal representative of the received electromagnetic waves by them feeding to a second multiplier (48) and multiplying it with a second sinusoidal signal of the same frequency as said second modulation signal, such as to generate a second demodulated signal,

(17.5) analysing said second demodulated signal.

**18.** Method according to claim 17, including the steps of

(18.1) selecting the same frequency for the first and second modulation signals,

(18.2) shifting the first and second modulation signals 90° in phase with respect to each other,

(18.3) selecting the first and second sinusoidal signals such that they are of the same frequency and 90° shifted in phase with respect to each other.

**19.** Method according to claim 17, wherein said electromagnetic waves are selected from the visible and/or the adjoining spectra of light, and the analysis of the first and second demodulated signals provides an indication of the oxygen saturation in the blood of a patient.

**20.** Method according to claim 18, including the steps of:

(20.1) Disabling the second modulation signal,

(20.2) applying a default phase $\varphi_{def}$ for demodulation,

(20.3) monitoring the first and second demodulated signals $L_1$ and $L_2$,

(20.4) calculating the correct system phase

$$\varphi = \varphi_{def} + \arctan(L_1/L_2),$$

(20.5) applying the correct system phase $\varphi$ for demodulation,

(20.6) enabling the second modulation signal.

FIG.1
PRIOR ART

FIG.2

FIG.3a

FIG.3b

FIG.3c

PRIOR ART

FIG. 4

$$f_{LED} - 2*f_{LINE}$$

$$3*f_{LINE} - f_{LED} \quad \ldots$$

$$5*f_{LINE} - 2*f_{LED}$$

FIG. 5

FIG.6

FIG. 7

FIG. 8

FIG.9

EP 0 760 223 A1

FIG. 10

EP 0 760 223 A1

FIG. 11

**European Patent Office**

**EUROPEAN SEARCH REPORT**

Application Number

EP 95 11 3654

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 502 717 (SENSORMEDICS CORP) 9 September 1992 * the whole document * | 1-5,7, 11-19 | A61B5/00 G01N21/31 |
| A | | 6,10 | |
| X | US-A-5 349 953 (MCCARTHY REX ET AL) 27 September 1994 * the whole document * | 1-5,7, 12-19 | |
| A | | 6,10,11 | |
| A | EP-A-0 102 816 (NELLCOR INC) 14 March 1984 * the whole document * | 1,2,8,10 | |

TECHNICAL FIELDS
SEARCHED      (Int.Cl.6)

A61B
G01N

The present search report has been drawn up for all claims

| Place of search | Date of completion of the search | Examiner |
|---|---|---|
| THE HAGUE | 23 January 1996 | Ferrigno, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document